# EUROPÄISCHE PATENTSCHRIFT

(11) **EP 1 074 586 B1**
(45) Veröffentlichungstag und Bekanntmachung des Hinweises auf die Patenterteilung: **19.05.2004**
(21) Anmeldenummer: 00114994.7
(22) Anmeldetag: 21.07.2000
(51) Int. Cl.: C09B 1/514, C07C 221/00

(54) **Verfahren zur Herstellung von Arylaminohydroxyanthrachinonen**
Process for the preparation of arylaminohydroxyanthraquinones
Procédé pour la préparation d'arylaminohydroxyanthraquinones

(30) Priorität: 02.08.1999 DE 19936282
(43) Veröffentlichungstag der Anmeldung: 07.02.2001
(73) Patentinhaber: Bayer Chemicals AG, 51368 Leverkusen (DE)
(72) Erfinder: Stawitz, Josef-Walter, Dr., 51519 Odenthal (DE); Michaelis, Stephan, Dr., 51519 Odenthal (DE)

(56) Entgegenhaltungen:
- DE-B- 1 278 631
- FR-A- 1 531 223
- FR-A- 1 573 136
- GB-A- 1 527 383

## Beschreibung

Die vorliegende Erfindung betrifft ein neues Verfahren zur Herstellung von Arylaminohydroxyanthrachinonen.

Die Kondensation von Arylaminen mit Chlorhydroxyanthrachinonen ist seit langem bekannt und wird z.B. in NL-A-6 504 570 und in GB-A-1 527 383 beschrieben. Diese Verfahren haben aber schwerwiegende Nachteile.

In NL-A-6 504 570 wird das Arylamin in hohem Überschuß und damit auch als Lösungsmittel benutzt. Dies führt zu hohen Amingehalten von über 1 % im Farbstoff, die eine Umlösung in einem anderen Lösungsmittel für viele Einsatzzwecke (Einfärbung von Kinderspielzeug, Lebensmittelverpackung usw.) zwingend erforderlich machen. Desweiteren entspricht die erhaltene Farbstoffqualität bezüglich der Brillanz nicht mehr den heutigen Anforderungen. Gemäß GB-A-1 527 383 werden Arylaminohydroxyanthrachinone aus Chlorhydroxyanthrachinonen mit Arylaminen in einem inerten Lösungsmittel (z.B. Ethoxyethanol) unter Verwendung von Borsäure und einem Überschuß Arylamin als Base hergestellt. Auch der aus diesem Verfahren erhaltene Farbstoff besitzt keine ausreichende Qualität und müßte weiteren Reinigungsschritten wie z.B. einer Umkristallisation unterzogen werden.

Aus DE-A-1 644 450 und DE-A 1 644 520 ist die Herstellung von Monoaminoanthrachinonen aus dem entsprechenden Monochlorvorläufer bekannt.

Es wurde nun ein Verfahren gefunden, welches es ermöglicht, Arylaminohydroxyanthrachinone in sehr guten Ausbeuten und in hoher Reinheit ohne die oben genannten Nachteile herzustellen.

Die vorliegende Erfindung betrifft ein Verfahren zur Herstellung von Arylaminohydroxyanthrachinonen der Formel (I) 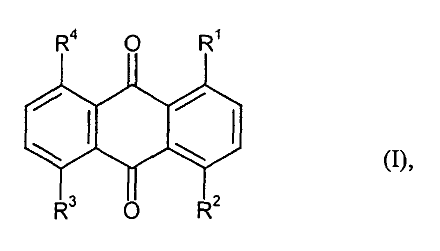 worin
- R¹, und R²,: unabhängig voneinander für NH-Ar stehen,
- R³ und R⁴: für Hydroxy stehen,
wobei
- Ar: für einen gegebenenfalls ein- oder mehrfachen, gleich oder verschieden substituierten Arylrest steht,
durch Umsetzung von dem entsprechenden Chlorhydroxyanthrachinon der Formel (II) 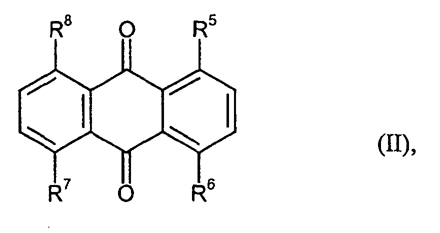 worin.
- R⁵ und R⁶: für Chlor stehen,
- R⁷ und R⁸: für Hydroxy stehen,
mit einem Amin der Formel (III)

H₂N-Ar (III),

worin
- Ar: die oben angegebene Bedeutung hat,
dadurch gekennzeichnet, dass pro Chloratom in der Verbindung der Formel (II) ein Mol Amin der Formel (III) zuzüglich eines Überschusses von 10 bis 100 % bezogen auf ein Mol Amin eingesetzt wird und die Umsetzung in Gegenwart einer Base, ausgewählt aus der Reihe der Phosphate der Alkalimetalle in einem inerten Lösungsmittel durchgeführt wird.

Das genannte Chlorhydroxyanthrachinon der Formel (II) wird im Allgemeinen in reiner Form für das erfindungsgemäße Verfahren eingesetzt. Allerdings ist es auch möglich, mit dem gleichen guten Ergebnis die Verbindung (II) als technische Ware einzusetzen.

Diese technische Ware enthält dann neben der Verbindung der Formel (II) im Allgemeinen noch 15 bis 25 % an Verunreinigungen in Form höher substituierter Produkte und/oder zu Formel (II) isomerer Produkte.

Bevorzugt wird für das erfindungsgemäße Verfahren 5,8-Dichlor-1,4-dihydroxyanthrachinon in Form dieser technischen Ware eingesetzt. Bei der erfindungsgemäßen Umsetzung mit dem Amin (III) werden auch die Chloratome der in der technischen Ware enthaltenen Nebenprodukte durch Aminogruppen ersetzt. Diese Reaktionsprodukte verbleiben allerdings in der Mutterlauge und können mit dieser von den erfindungsgemäß hergestellten Produkten (I) abgetrennt werden und stellen somit keine Beeinträchtigung für die Qualität des Endproduktes dar.

Insbesondere handelt es sich in der Definition von Ar um einen Naphthylrest, welcher gegebenenfalls ein- oder mehrfach, gleich oder verschieden durch Alkyl und/oder Halogen substituiert ist, oder bevorzugt um einen Phenylrest, welcher gegebenenfalls ein- oder mehrfach, gleichen oder verschiedenen substituiert ist, wobei als Substituenten in Frage kommen Cyano, Carboxy, Nitro, Alkylsulfonyl, Carbonyl, Sulfo, Alkylaminocarbonyl, Alkylcarbonylamino, Alkyl, Cycloalkyl, Alkoxy, Trifluormethyl, Halogen und Aryl, das seinerseits wiederum substituiert sein kann durch die oben für Naphthyl und Phenyl genannten Reste.

Unter Alkyl, auch in den zusammengesetzten Begriffen wie Alkylsulfonyl, Alkylaminocarbonyl und Alkylcarbonylamin, ist insbesondere geradkettiges oder verzweigtes C₁- bis C₆-Alkyl zu verstehen. Cycloalkyl steht insbesondere für Cyclopentyl oder Cyclohexyl. Unter Alkoxy ist bevorzugt geradkettiges oder verzweigtes C₁- bis C₆-Alkoxy zu verstehen. Aryl steht insbesondere für Phenyl oder Naphthyl. Halogen steht bevorzugt für Chlor oder Brom.

Bevorzugte Substituenten für Phenyl und Naphthyl sind Methyl, Ethyl, n-Propyl, iso-Propyl, n-Butyl, sec-Butyl, tert-Butyl, Cyclohexyl, Methoxy, Chlor, Brom und Phenyl, das seinerseits substituiert sein kann. Besonders bevorzugt ist Methyl, tert-Butyl, n-Butyl, Cyclohexyl und Phenyl.

Die Arylamine (III) werden in der theoretisch erforderlichen Menge zuzüglich eines Überschusses von vorzugsweise 10 bis 50 %, besonders bevorzugt 10 bis 30 %, insbesondere bevorzugt 10 bis 20 %, bezogen auf 1 Mol Arylamin, eingesetzt.

Als bevorzugt eingesetzte inerte Lösungsmittel seien genannt:
N-Methylpyrrolidon, Chlorbenzol, 1,2-Dichlorbenzol, 1,2,4-Trichlorbenzol, Xylol, Nitrobenzol und Phenol.

Besonders bevorzugt sind N-Methylpyrrolidon und Dichlorbenzol.

Bevorzugte Basen sind Trinatriumphosphat, Dinatriumhydrogenphosphat und Natriumdihydrogenphosphat.

Besonders bevorzugt sind Dinatriumhydrogenphosphat und Trinatriumphosphat. Es werden im Allgemeinen 0,5 bis 2,5 Äquivalente an Base, bevorzugt 0,5 bis 1 Äquivalent pro in den Verbindungen der Formel (II) auszutauschenden Chlor-Atome bzw. pro Mol freiwerdende Salzsäure eingesetzt.

Die Reaktion wird ausgeführt bei einer Temperatur zwischen 80° und 200°C, bevorzugt zwischen 110° und 190°C.

Die Verunreinigungen bleiben in der Mutterlauge gelöst und werden bei der Isolierung mittels Filtration und Waschen des Rückstandes durch Verdrängung der Mutterlauge praktisch vollständig entfernt.

Das erfindungsgemäße Verfahren liefert die Arylaminohydroxyanthrachinone in sehr guter Ausbeute von 80 bis 100 %, vorzugsweise >90 %, dabei beträgt der Gehalt an Verunreinigungen durch Amin (III) weniger als 600 ppm, vorzugsweise weniger als 300 ppm.

Die Verbindungen der Formel (I) stellen wertvolle Farbstoffe zum transparenten und gedeckten Färben von Kunststoffen wie Polystyrol, Polyamid, Polycarbonat, Polyethylen, Polyester, z.B. Polyethylenterephtalat oder Polyethylenbutylat, ABS und ABS-PC-Blends dar. Sie werden sowohl zur Massefärbung als auch zur Spinnfärbung eingesetzt.

### Beispiele

### Beispiel 1

Eine Mischung aus 93,0 Teilen 5,8-Dichlor-1,4-dihydroxyanthrachinon (technische Ware, 80 %ig), 300 Teilen 1,2-Dichlorbenzol, 108 Teilen p-tert-Butylanilin und 22,5 Teilen Dinatriumhydrogenphosphat wird in einer Stunde unter Stickstoff auf 150°C erwärmt und eine Stunde bei dieser Temperatur gehalten.

Anschließend heizt man in einer Stunde auf 170°C und hält zwei Stunden bei dieser Temperatur. Dann heizt man auf 180°C und hält diese Temperatur 12 Stunden. Es wird auf 90°C gekühlt und innerhalb von 1 h 375 Teile Methanol so zugetropft, dass der Ansatz bei 65 bis 68°C gehalten wird. Der Ansatz wird bei Rückfluß eine Stunde nachgerührt, auf 25°C abgekühlt, zwei Stunden bei 25°C nachgerührt und abgesaugt.

Der Farbstoff wird mit 270 Teilen einer 2:1-Mischung aus Methanol und Dichlorbenzol, anschließend mit 375 Teilen heißem Methanol (65°C) und dann mit 1 000 Teilen Wasser (80°C) gewaschen. Das feuchte Produkt wird bei 70°C im Vakuum getrocknet und man erhält 126,8 Teile des Farbstoffs der folgenden Formel 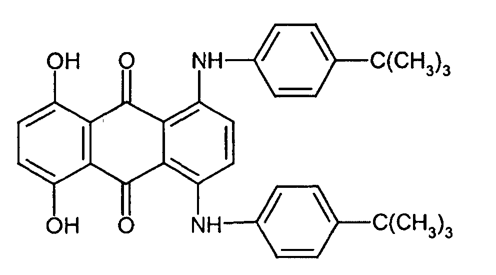 das sind 98,6 % d. Th., bezogen auf die Hauptkomponente des eingesetzten 5,8-Dichlor-1,4-dihydroxyanthrachinons.

Der Farbstoff enthält <100 ppm p-tert-Butylanilin.

### Beispiel 2

Eine Mischung aus 93,0 Teilen 5,8-Dichlor-1,4-dihydroxyanthrachinon (technische Ware, 80 %ig), 250 Teilen N-Methylpyrolidon, 112 Teilen p-tert-Butylanilin und 85,2 Teilen Dinatriumhydrogenphosphat wird in einer Stunde unter Stickstoff auf 150°C erwärmt und drei Stunden bei dieser Temperatur gehalten.

Anschließend heizt man in einer Stunde auf 180°C und hält 12 Stunden bei dieser Temperatur. Es wird auf 120°C gekühlt und innerhalb von 1 h 375 Teile Methanol so zugetropft, dass der Ansatz bei 65 bis 68°C gehalten wird. Der Ansatz wird bei Rückfluß eine Stunde nachgerührt, auf 55°C abgekühlt, zwei Stunden bei 55°C nachgerührt und abgesaugt.

Der Nutschkuchen wird mit 115 Teilen einer 2:3 Mischung aus Methanol und N-Methylpyrrolidon zur Verdrängung der Mutterlauge gewaschen. Anschließend wird mit 1 000 Teilen heißem Methanol (65°C) und 1 000 Teilen Wasser (80°C) gewaschen. Das feuchte Produkt wird bei 70°C im Vakuum getrocknet und man erhält 120,6 Teile des reinen Farbstoffes, das sind 93,8 % d. Theorie.

Der Farbstoff enthält <50 ppm p-tert.-Butylanilin.

## Patentansprüche

1. Verfahren zur Herstellung von Arylaminohydroxyanthrachinonen der Formel (I) 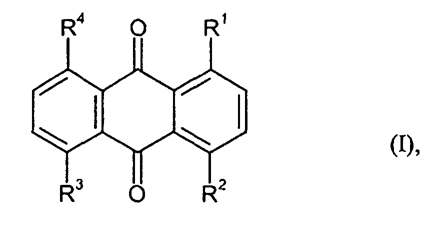 worin
R¹, und R², unabhängig voneinander für NH-Ar stehen,
R³ und R⁴ für Hydroxy stehen,
wobei
Ar für einen gegebenenfalls ein- oder mehrfachen, gleich oder verschieden substituierten Arylrest steht,
durch Umsetzung von dem entsprechenden Chlorhydroxyanthrachinon der Formel (II) 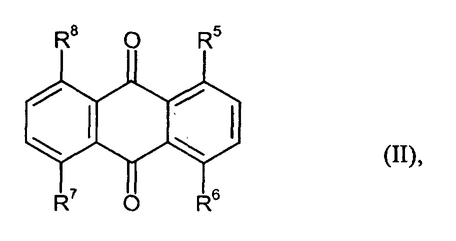 worin
R⁵, und R⁶ für Chlor stehen und
R⁷ und R⁸ für Hydroxy stehen,
mit einem Amin der Formel (III)
H₂N-Ar (III),
worin
Ar die oben angegebene Bedeutung hat,
**dadurch gekennzeichnet, dass** pro Chloratom in der Verbindung der Formel (II) ein Mol Amin der Formel (III) zuzüglich eines Überschusses von 10 bis 100 % bezogen auf ein Mol Amin eingesetzt wird und die Umsetzung in Gegenwart einer Base, ausgewählt aus der Reihe der Phosphate der Alkalimetalle, in einem inerten Lösungsmittel durchgeführt wird.

2. Verfahren gemäß Anspruch 1, **dadurch gekennzeichnet, dass** als Amin eine Verbindung der Formel (III) eingesetzt wird, worin Ar für gegebenenfalls einoder mehrfach, gleich oder verschieden durch Alkyl und/oder Halogen substituiertes Naphthyl oder für gegebenenfalls ein- oder mehrfach gleich oder verschieden durch Cyano, Carboxy, Nitro, Alkylsulfonyl, Carbonyl, Sulfo, Alkylaminocarbonyl, Alkylcarbonylamino, Alkyl, Cycloalkyl, Alkoxy, Trifluormethyl, Halogen und Aryl, das seinerseits wiederum substituiert sein kann durch die oben für Naphthyl und Phenyl genannten Reste, substituiertes Phenyl steht.

3. Verfahren gemäß Ansprüchen 1 bis 2, **dadurch gekennzeichnet, dass** als Amin (II) p-tert.-Butylanilin eingesetzt wird.

4. Verfahren gemäß Ansprüchen 1 bis 3, **dadurch gekennzeichnet, dass** als Base Dinatriumhydrogenphosphat oder Trinatriumphosphat eingesetzt wird.

5. Verfahren gemäß Ansprüchen 1 bis 4, **dadurch gekennzeichnet, dass** ein Lösungsmittel aus der Reihe N-Methylpyrrolidon, Chlorbenzol, Dichlorbenzol, 1,2,4-Trichlorbenzol, Xylol, Nitrobenzol und Phenol eingesetzt wird.

6. Verfahren gemäß Ansprüchen 1 bis 5, **dadurch gekennzeichnet, dass** die Umsetzung bei einer Temperatur zwischen 80° und 200°C durchgeführt wird.

7. Verfahren gemäß Ansprüchen 1 bis 6, **dadurch gekennzeichnet, dass** 1 Mol 5,8-Dichlor-1,4-dihydroxyanthrachinon mit 2,4 Mol p-tert.-Butylanilin in Gegenwart von Dinatriumhydrogenphosphat in 1,2-Dichlorbenzol oder N-Methylpyrrolidon umgesetzt wird.

## Claims

1. Process for preparing arylaminohydroxyanthraquinones of the formula (I) 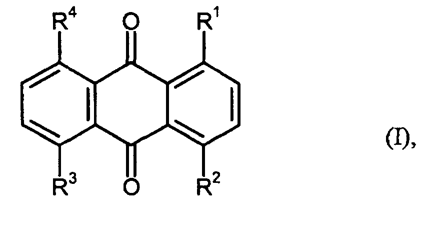 where
R¹ and R² are independently NH-Ar,
R³ and R⁴ are hydroxyl,
where
Ar is an aryl radical optionally substituted by one or more identical or different substituents,
by reacting the appropriate chlorohydroxyanthraquinone of the formula (In 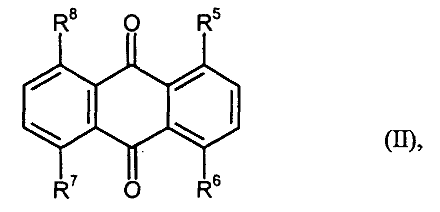 where
R⁵ and R⁶ are chlorine and
R⁷ and R⁸ are hydroxyl,
with an amine of the formula (III)
H₂N-Ar (III),
where
Ar is as defined above,
**characterized in that** one mole of amine of the formula (III) plus an excess of 10 to 100% based on one mole of amine is used per chlorine atom in the compound of the formula (II) and the reaction is carried out in an inert solvent in the presence of a base selected from the group consisting of the phosphates of the alkali metals.

2. Process according to Claims 1, **characterized in that** the amine used is a compound of the formula (III) where Ar is naphthyl optionally substituted by one or more identical or different substituents selected from the group consisting of alkyl and halogen or is phenyl optionally substituted by one or more identical or different substituents selected from the group consisting of cyano, carboxyl, nitro, alkylsulphonyl, carbonyl, sulfo, alkylaminocarbonyl, alkylcarbonylamino, alkyl, cycloalkyl, alkoxy, trifluoromethyl, halogen and aryl, which in turn may be substituted by the radicals mentioned above for naphthyl and phenyl.

3. Process according to Claims 1 to 2, **characterized in that** amine (II) is p-tert-butylaniline.

4. Process according to Claims 1 to 3, **characterized in that** the base used is disodium hydrogenphosphate or trisodium phosphate.

5. Process according to Claims 1 to 4, **characterized in that** the solvent used is selected from the group consisting of N-methylpyrrolidone, chlorobenzene, dichlorobenzene, 1,2,4-trichlorobenzene, xylene, nitrobenzene and phenol.

6. Process according to Claims 1 to 5, **characterized in that** the reaction is carried out at a temperature between 80° and 200°C.

7. Process according to Claims 1 to 6, **characterized in that** 1 mol of 5,8-dichloro-1,4-dihydroxyanthraquinone is reacted with 2.4 mol of p-tert-butylaniline in 1,2-dichlorobenzene or N-methylpyrrolidone in the presence of disodium hydrogenphosphate.

## Revendications

1. Procédé pour la préparation des arylaminohydroxyanthraquinones de formule (I) 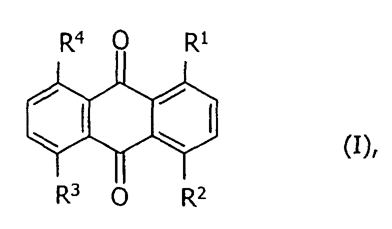 dans laquelle
R¹ et R² représentent chacun, indépendamment l'un de l'autre, un groupe NH-Ar,
R³ et R⁴ représentent des groupes hydroxy,
Ar représente un radical aryle portant le cas échéant un ou plusieurs substituants identiques ou différents,
par réaction de la chlorhydroxyanthraquinone correspondante de formule (II) 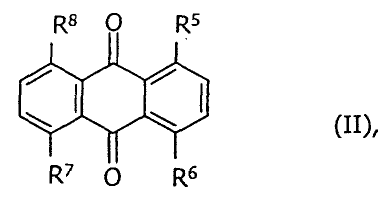 dans laquelle
R⁵ et R⁶ représentent le chlore,
R⁷ et R⁸ des groupes hydroxy,
avec une amine de formule (III)
H₂N-Ar (III),
dans laquelle
Ar a les significations indiquées ci-dessus,
**caractérisé en ce que**, pour 1 atome de chlore du composé de formule (II), on met en oeuvre 1 mol de l'amine de formule (III) plus un excès de 10 à 100 % par mole d'amine et on effectue la réaction en présence d'une base choisie dans la classe des phosphates de métaux alcalins, dans un solvant inerte.

2. Procédé selon la revendication 1, **caractérisé en ce que** l'on utilise en tant qu'amine un composé de formule (III) dans laquelle Ar représente un radical naphtyle portant éventuellement un ou plusieurs substituants identiques ou différents choisis parmi les groupes alkyle et/ou les halogènes ou un radical phényle portant éventuellement un ou plusieurs substituants identiques ou différents choisis parmi les groupes cyano, carboxy, nitro, alkylsulfonyle, carbonyle, sulfo, alkylaminocarbonyle, alkylcarbonylamino, alkyle, cycloalkyle, alcoxy, trifluorométhyle, les halogènes et les groupes aryle, ces derniers pouvant eux-mêmes porter les substituants mentionnés ci-dessus en référence aux radicaux naphtyle et phényle.

3. Procédé selon les revendications 1 à 2, **caractérisé en ce que** l'amine (II) mise en oeuvre est la p-tert-butylaniline.

4. Procédé selon les revendications 1 à 3, **caractérisé en ce que** la base mise en oeuvre est l'hydrogénophosphate disodique ou le phosphate trisodique.

5. Procédé selon les revendications 1 à 4, **caractérisé en ce que** l'on met en oeuvre un solvant choisi parmi la N-méthylpyrrolidone, le chlorobenzène, le dichlorobenzène, le 1,2,4-trichlorobenzène, le xylène, le nitrobenzène et le phénol.

6. Procédé selon les revendications 1 à 5, **caractérisé en ce que** la réaction est réalisée à une température de 80 à 200°C.

7. Procédé selon les revendications 1 à 6, **caractérisé en ce que** l'on fait réagir 1 mol de 5,8-dichloro-1,4-dihydroxyanthraquinone avec 2,4 mol de p-tert-butylaniline en présence d'hydrogénophosphate disodique dans le 1,2-dichlorobenzène ou la N-méthylpyrrolidone.
